# EUROPEAN PATENT APPLICATION

(11) **EP 0 224 594 A1**
(43) Date of publication of application: **10.06.1987**
(21) Application number: 86903571.7
(22) Date of filing: 27.05.1986
(51) Int. Cl.: A61F 13/20

(54) **TAMPON**

(30) Priority: 27.05.1985 JP 77651/85 U
(71) Applicant: SHIMATANI, Kazuo, Koza-gun, Kanagawa 253-01 (JP)
(72) Inventor: SHIMATANI, Kazuo, Koza-gun, Kanagawa 253-01 (JP)
(74) Representative: Noz, Franciscus Xaverius, Ir.
(86) International application number: JP8600269
(87) International publication number: WO8606956

(57) **Abstract**

A sanitary tampon formed in the shape of a bullet by subjecting a tampon body, which is formed of a highly water- absorbent material to a belt-like structure, to a fluff- preventing surface treatment, fixing a puller string to the tampon body, subjecting the resultant tampon body to a mild, primary compressing operation to obtain a ring-shaped product having a plurality of folds at least on the inner circumferential surface thereof, and compressing the ring-shaped tampon again from the outer circumferential surface thereof so as to produce a bullet-like tampon body. This tampon is adapted to expand radially when it absorbs the menstrual blood, and thereby abuts on the vaginal wall surface, so that the menstrual blood flowing along the vaginal wall surface can be surely absorbed. When the puller string is drawn, the ring-shaped tampon body is unfolded, and comes out in a belt-shaped state. Therefore, this tampon can be taken out easily.

## Description

### Field of the Invention

The present invetion relates to a sanitary tampon surely absorbing menstrual discharge without leaking out and is easily taken out of body after use.

Several types of sanitary tampons have been used to menstruation and the like. However, these tampons are devided roughly into two groups. The one of them is one prepared by forming a wider belt of an absorbent such as an absorbent cotton, an absorbent paper and the like, winding tightly the belt type mass in a whirl form so as not to come loose, and adding pressure to the whirl type mass from outer circomference to obtain a bullet like tampon. The other one is formed by compressing, as it is, the above mentioned belt like absorbent from top and bottom, and from horizontal direction to obtain a stick like tampon. These conventional tampons can be esily inserted into vagina, because they are formed by compression-shaping and adding several additional components.

However, these conventional tampons have basically the problems shown in the folllowing. In regard to the former type tampon, they absorb menstrual blood well, but they absorb the menstrual blood falling down along the vagina wall to swell in the directon of radii. After use, they have to be pulled out of the body in the directio-3of longitude under a condition swelled and expanded into large diameter. Therefore, it is not easy to take the swelled tampon out of body. Furthermore, it is often happened to lend a doctor's hand to take out a used tampon, since a string provided with a tampon for taking out of the body (hereinafter refers to as string) is cut down on the way.

On the other hand, the latter type of tampon swells and expands in the direction of up-and-down by absorbing menstrual blood and thus the swelled one is taken out of the body with easier than the former one, but this type of tampon has defects that a gap is formed between vagina wall and the tampon when in use and the tampon can not sufficiently absorb blood.

### Summary of the Invention

Therefore, the object of the present invention is to provide a tampon which surely absorbs menstrual blood irrespetive of the quantity thereof, and is easily taken out of the body.

The sanitary tampon of the inventin is preoared by a process that a tampon body mass made of a soft substance rich in water-absorbable material is formed to a belt like shape and the belt like mass is subjected to a treatment for peventing the tampon from raising nap and a string for taking out the tampon out of the body is attached to the treated mass and a primary compression is added to the obtained tampon body to get a ring shaped tampon body having a number of pleats at least on the inner surface of the tampon body in the direction of width and thus obtained ring formed tampon body is recompressed from outer circumference to obtain a bullet type tampom, the tampon of the invention which surely absorbs menstrual blood irrespective of the quantity thereof and is easily taken out of the body.

### Brief Description of the Drawings

The drawings relate to a sanitary tampon and the like of the invention.
Fig 1 shows a general perspective view of the tampon of the invention.
Fig 2 is a cross-sectional view of the tampon shown on Fig 1.
Fig 3 is an explanatory drawing showing a procedure to form the tampon body having ring like shape.
Fig 4 is a perspective view of the tampon body having ring like shape.
Fig 5 is a perspective view of the tampon prepared by compression-shaping the tampon body having ring like shape.
Fig 6 to 9 is explanatory drawings successively showing the states of the tampon taking out after use.

### Detailed Description of the Preferred Embodiments

The tampon of the invention is prepared by that the tampon body having a belt like shape is formed into a ring shaped tampon body having many pleats at least on the inner surface thereof in the direction of width and then pressure is added to the ring shaped tampon body from outer circumference to obtain a tampon having bullet like shape, the tampon of the invention. Therefore, the tampon of the invention swells in the direction of radii by absorbing menstrual blood to be enlarged and rstored to a ring shaped tampon. At this state, both ends of the tampon is enforced to open and enlarge the gap of the both ends of the tampon by a force streching the pleats on the inner and outer surface and softly contact with the inner surface of vagina. Therefore, the tampon can surely absorb the menstrual blood falling down along the vagina wall.

Then, when the string provided on the tampon is pulled, the tampon swelled in a form of ring shape in the vagina is pulled down holding below the side end of the tampon to which the string is attached, to collapse its ring like shape and store the original belt like shape and the tampon ia taken out in the said belt like shape. Therefore, the tampon is easily and safely pulled out of the body with a weak force.

Accordingly, even a user having a narrow vagina can use a tampon having a large volume, for example a tampon having a twice volume of an ordinary tampon, that is a tampon having twice capacity of absorbability, and at that time the outer surface of the tampon is fitted well to the inner walli of vagina to be able to surely absorb menstrual blood flown downm with an effect that twice and more amounts of menstrual discharge can be absorbed.

Furthermore, though the present tampon is pulled out in a form of a belt, the tampon has no danger to remain flue in the vagina, since the front and back surface of the tampon body is subjected to a treatment for preventing from raising nap.

In the following, the invention will be explained in detail on the basis of an example shown on the drawings.

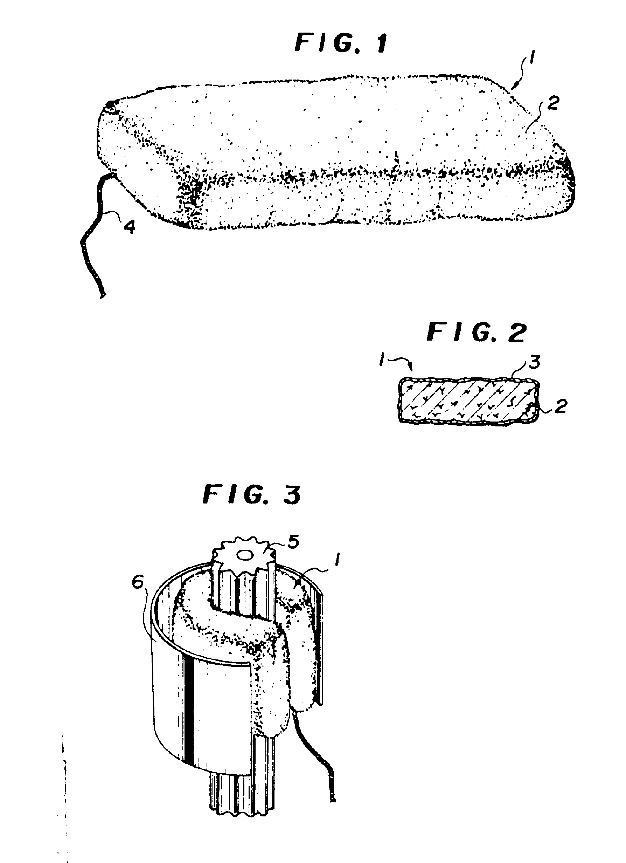

Fig 1 and 2 show a tampon body 1 relating to an example of the invention. In the drawings, 2 shows an absobent constructing tampon body 1, which is made of a material having a high absorbability such as sanitary cotton, absorbent paper etc. or a material mixing these materials with polymer absorbent. For example, in a case of regular size, a soft slender mass having a diameter of about 4 cm and a length of about 12 cm is slightly pressed from the surface to be flattened into a rectangular parallelopiped having a thickness of 1 cm, a width of 4 cm and a length of about 12 cm which is somewhat thicker than the ordinary one.

3 is an outer cover, which covers the outer surface of the absorbent, has a small pouch like shape and is used to prevent the tampon from raising nap. The outer cover is made of a thin gauze like cloth weaved in, using a water soluble material as a part of woof and cotton yarn as warf. An open side end of the outer cover itself made somewhat longer than the absorbent 2 forms the string 4 by twisting the exessive part of the outer cover. As another embodiment, the outer cover 3 may be a sheet like material. The sheet like material covers the front and back surfaces of absorbent 2. At the time, the string may be separately attached to the tampon body. Since the outer cover 3 itself is used to prevent tampon body from raising nap the surface of absorbent 2. A sheet like materials used as an outer covering in conventional one to cover the surface of the products after compression may also be utilized to the outer cover of the invention.

The tampon body 1 is, as shown on Fig 3, wound around a winding core which has many tooth on the outer surface and an outer circumference longer than that of the tampon body, and then the tampon body is fastened tightly from outside with band 6 having a wider width. While the pleats having a wide pitch are formed on the inner surface of the tampon body in the direction of width, and a small rumples are made on the outer surface, the tampon body is subjected to primary compression into a ring form to obtain a mass (tampon body 1') somewhat compressed in the direction of thickness. However, of course, for forming the above mentioned pleats on the tampon body, other publicly known several means may be also used to the invention.

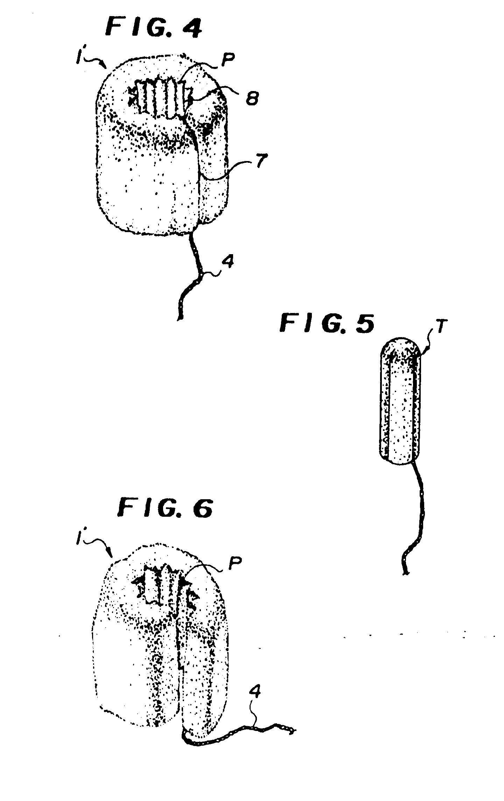

Thus prepared ring form tampon body 1' has, as shown on Fig 4, slightly opened gap 7 between the both ends I'a and l'a. To the inner part is provided with void parts 8.

Then, the tampon of the invention is formed in a bullet like shape as shown on Fig 5 by gradualy adding pressure to the ring shaped tampon body 1' from an arbitrary direction of the outer circumference, for example from 8 diections equally devided in the direction of radii to compress the ring shaped tampon body 1' into the bullet like shape.

The above mentioned tampon inserted in vagina with finger or applicator (not shown) absorbs the menstrual blood to swell and enlarge in radial dirction to return to the state of ring like formed tampon body 1', that is, the form prior to the commpression as shown on Fig 4. In this state, because pleats are formed on the inner circumference of the tampon, the pleats have a restoring force in some degree in the direction of elongating these pleats, namely in a direction of spreading and opening the both ends l'a and l'a. Accordingly, even if the inserted tampon T were not located on the center of vagina, the outer circumference thereof would softly contact with the inner wall of vagina and thus the tampon surely absorbs the menstrual blood fallen down along the inner wall surface of vagina. It does well that the pleats are provided on a surfacce giving in some degree a restoring force in the direction that the ring like tampon body 1' is spreaded and opened, when the said pleats are lengthened by absorbing blood.

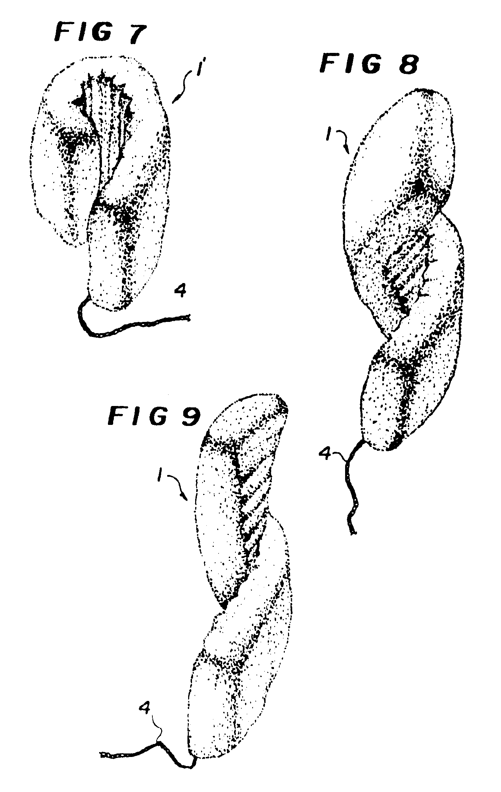

As shown in Fig 6 to 9, when the string is pulled in order to take out of the tampon after a definit time has passed, one end of the ring form tampon body 1' to which the string is attached is slipped out downwardly to deform the ring shape of the tampon body 1' (Fig 6 and 7) and is radually taken out to the exterior from the pulled end of 1'a (Fig 8) in a manner rubbing the inner wall of vagina and finallly the tampon is taken out in a form of an ori ginal belt like shape mentioned in the above.

The tampon of the invention can be easily taken out to the exterior with a lesser force than the force needed to the conventional one in which the tampon is pulled out in a state of swelled cylinder as it is.

In addition. when the tampon of the invention absorbs menstrual blood in vagina, a part of woof of the outer cover 3 is solved in blood and the surface of the tampon is wetted in a gel state to make smooth the taking out of the tampon after use.

Furthermore, although the tampon body 1 is taken out in a state of restoring such an original shape as belt, there is no possibility to remain cotton yards and the like in vagina, because the tampon body 1 is covered by outer cover 3 and is added a surface treatment for preventing the tampon body from raising nap.

In the above mentioned example, the string is made utlizing an end part of the outer cover provided for preventing from fluffing of the front and back surface of the tampon body 1. Therefore, this method saves trouble. Futhermore, the woof of the string 4 is made of water-soluble material and the warf is made of cotten. Thus, the string can fully withstand the tenssion given to the string.

The absorbing amounts of the menstrual blood to a tampon depends on the volume of tampon body. Several type of tampons can be prepared according to much or less of the amounts of menstrual blood by suitably increasing or decreasing the length, width or thickness of the tampon body. In any way, since the diameter of a tampon becomes minimum at the time of taking out, the tampon of the invention is easily taken out.

It will be appreciated that the instant specification and examples are set force by way of illustration and no limitation, and various modifications and changes may be made without departing from the spirit and scope of the present invention.

## Claims

1. A sanitaory tampon which is characterized by that a tampon body mass made of a soft substance rich in water-absorbable mate- rialis is formed to a belt like shape and the belt like mass is subjected to a treatment for peventing the tampon from raising nap and a string for taking the tampon out of the body is attached to the treated mass and a primary compression is added to the obtained tampon body to get a ring shaped tampon body having a number of pleats on the inner surface of the tampon body in the direction of width and thus obtained ring formed tampon body is recompressed from outer circumference to obtain a bullet like sanitary tampom.

2. A sanitory tampon according to claim 1, wherein said tampon body is obtained by covering a absorbent made of such a material as sanitory cotton, absorbent paper and the like with an outer cover having a small pouch like shape which is made of a gauze like cloth containing as a part of woof a water-soluble polymer such as polyethylene-glycol and the like.

3. A sanitory tampon according to claim 1 or 2, wherein said string funished to the tampon for taking the tampon out of the body is prepared by intertwisting one side end of the oped side of the outer cover which is pepared by weaving a yarn made of said polymer as a part of woof and a yarn such as coton as warp.
